(19) **Europäisches Patentamt / European Patent Office / Office européen des brevets**

(11) **EP 3 295 342 B1**

(12) **EUROPEAN PATENT SPECIFICATION**

(45) Date of publication and mention
of the grant of the patent:
**25.08.2021  Bulletin 2021/34**

(51) Int Cl.:
***G16H 20/10*** (2018.01)

(21) Application number: **16725237.8**

(86) International application number:
**PCT/IB2016/052755**

(22) Date of filing: **12.05.2016**

(87) International publication number:
**WO 2016/181351 (17.11.2016 Gazette 2016/46)**

(54) **BLOOD GLUCOSE MANAGEMENT DEVICE FOR CALCULATING BOLUS INSULIN**

GERÄT FÜR BLUTZUCKERMANAGEMENT ZUR BERECHNUNG DER INSULINABGABE

APPAREIL DETERMINANT LE DOSE D'INSULINE POUR LA GESTION DE GLUCOSE SANGUIN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.05.2015  US 201562160892 P**

(43) Date of publication of application:
**21.03.2018  Bulletin 2018/12**

(73) Proprietor: **Ascensia Diabetes Care Holdings AG
4052 Basel (CH)**

(72) Inventors:
- **GASS, Jennifer, L.**
  **Tarrytown, New York 10591 (US)**
- **MORIN, Robert, W.**
  **Boca Raton, Florida 33496 (US)**

(74) Representative: **Gille Hrabal
Partnerschaftsgesellschaft mbB
Patentanwälte
Brucknerstraße 20
40593 Düsseldorf (DE)**

(56) References cited:
**US-A1- 2009 253 970     US-A1- 2012 245 447**

- **John Walsh ET AL: "Guidelines for Optimal Bolus Calculator Settings in Adults", Journal of Diabetes Science and Technology Volume Diabetes Technology Society J Diabetes Sci Technol, 1 January 2011 (2011-01-01), pages 129-135, XP055293839, Retrieved from the Internet: URL:http://www.ncbi.nlm.nih.gov/pmc/articl es/PMC3045234/pdf/dst-05-129.pdf [retrieved on 2016-08-05]**
- **Maguire, Phyllis: "Tips for calculating a total daily dose of insulin", Today'sHospitalist , 2 April 2015 (2015-04-02), page 4PP, XP002760585, Retrieved from the Internet: URL:https://web.archive.org/web/2015040200 5842/http://www.todayshospitalist.com/arti cles/tips-for-calculating-a-daily-dose-of- insulin.htm [retrieved on 2016-08-05]**

**Description**

TECHNICAL FIELD

The present disclosure relates generally to an apparatus for assisting in determining a carbohydrate factor and a correction factor used for determining an amount of bolus insulin required to maintain or return a user's blood glucose to a desired range.

BACKGROUND

The quantitative determination of analytes in body fluids is of great importance in the diagnosis and maintenance of certain physiological conditions. For example, individuals with diabetes frequently check the glucose level in their bodily fluids. The results of such tests can be used to regulate the glucose intake in their diets and/or to determine whether insulin or other medication needs to be administered.

Diagnostic systems, such as blood-glucose monitoring systems, may employ an instrument, such as a meter, to calculate the glucose concentration value in a fluid sample from an individual. Such instruments operate by measuring an output, such as current or light, from a reaction with the glucose in the sample. The test results typically are displayed and stored by the meter. Basic systems allow the user to access the test results directly from the meter via a display and a keypad or other interactive component.

Bolus dosing algorithms or calculators, often included in existing more sophisticated instruments, assist diabetic individuals in determining the amount of bolus insulin needed to return their blood glucose levels to, or maintain within, a desired range. To accurately calculate the bolus dosage, two types of information are generally required to be input into the instrument: (1) short-term parameters that depend on the individual's current carbohydrate level, exercise status, and insulin levels; and (2) user-specific parameters that describe how the individual's body processes carbohydrates, exercise, and insulin. User-specific parameters may include a carbohydrate factor, an exercise adjustment factor, and a correction factor. The carbohydrate factor, sometimes known as a carb-to-insulin ratio, refers to the amount of carbohydrates affected by one unit of insulin. The correction factor, sometimes referred to as insulin sensitivity, is the amount a user's blood glucose level drops per one unit of insulin.

The user-specific parameters (e.g., the carbohydrate factor and the correction factor) may be determined either by examining the individual's blood glucose response over time or by estimating using a series of equations. These equations can, for example, estimate the carbohydrate factor and the correction factor using a combination of the individual's weight and diabetes type, whether the individual uses an insulin pump, and his or her daily dose of insulin.

The user-specific parameters are generally defined by the individual during, for example, an initial bolus calculator setup. If the values associated with the individual's user-specific parameters are known, the values may be entered directly into the instrument. However, if the individual does not know these values, they must be calculated. If the equations that can be used to calculate the user-specific parameter values are known, a calculator - or at least a paper and pencil to assist with the math - is often needed to do the calculations. If the equations are not known, they generally need to be looked up externally. Thus, existing methods of calculating user-specific parameters can be time consuming, inconvenient, and prone to error.

It is referred to the documents US2012245447A1 and US2009253970A1 as well as John Walsh ET AL, "Guidelines for Optimal Bolus Calculator Settings in Adults", Journal of Diabetes Science and Technology Volume Diabetes Technology Society J Diabetes Sci Technol, (20110101), pages 129 - 135, URL: http://www.ncbi.nlm.nih.gov/pmc/articles/PMC3045234/pdf/dst-05-129.pdf, (20160805), XP055293839 [Y] 1-21 * pgs. 131, 134, 135 *, and Maguire, Phyllis, "Tips for calculating a total daily dose of insulin", (20150402), page 4PP, Today's Hospitalist, URL: https://web.archive.org/web/20150402005842/http://www.todayshospitalist.com/articles/tips-for-calculating-a-daily-dose-of-insulin.htm, (20160805), XP002760585 [Y] 1-21 * page 2 - page 3.

Thus, there is a need for improvements that help simplify bolus calculations.

SUMMARY

The problem is solved by a glucose management device according to claim 1. Also disclosed, yet not claimed, is a method of operating a glucose management device. The glucose management device includes one or more processing components, a housing, a user interface including a display and a user-input mechanism, and a memory. The method comprises displaying on the display a prompt for initiating a carbohydrate factor determination sequence via the user-input mechanism and displaying on the display at least one query, the at least one query including a query associated with a user's type of diabetes. The method further comprises receiving the user's response to the at least one query via the user-input mechanism and determining, via the at least one processing component, the carbohydrate factor using the user's response to the at least one query and information stored in the memory.

Also disclosed is a method of operating a glucose management device. The glucose management device includes one or more processing components, a housing, a user interface including a display and a user-input mechanism, and a memory. The method comprises displaying on the display a prompt for initiating a correction factor determination sequence via the user-input mechanism and displaying on the display at least one query, the at least one query being including a query associated with a user's type of diabetes and a query associated with the user's insulin pump usage. The method further comprises receiving the user's response to the at least one query via the user-input mechanism and determining, via the at least one processing component, the correction factor using the user's response to the at least one query and information stored in the memory.

Also disclosed is a glucose management device for managing a user's blood glucose level is disclosed. The glucose management device comprises a housing, a user interface including a display and a user-input mechanism, a memory device for storing programmed instructions, and a processing component, the processing component configured to cause the display to display a prompt for initiating a carbohydrate factor determination sequence or a correction factor determination sequence in response to input received via the user-input mechanism. The processing component is further configured to cause the display to display at least one query, the at least one query being associated with a user's type of diabetes, the user's weight, the user's total daily dose of insulin, whether the user uses an insulin pump, or combinations thereof. The processing component is further configured to receive the user's response to the at least one query via the user-input mechanism and determine the carbohydrate factor or the correction factor using the user's response to the at least one query and information stored in the memory device.

Still other aspects, features, and advantages of the present invention are readily apparent from the following detailed description, by illustrating a number of exemplary embodiments and implementations, including the best mode contemplated for carrying out the present invention. The present invention is also capable of other and different embodiments, and its several details can be modified in various respects, all without departing from the spirit and scope of the present invention. Accordingly, the drawings and descriptions are to be regarded as illustrative in nature, and not as restrictive. The invention is to cover all modifications, equivalents, and alternatives falling within the scope of the claims.

## BRIEF DESCRIPTION OF THE DRAWINGS

Exemplary embodiments are illustrated in referenced figures. It is intended that the embodiments and figures disclosed herein are to be considered illustrative rather than restrictive.

FIG. 1 illustrates an exemplary system, including an exemplary meter, which is described to demonstrate aspects of the present invention.
FIG. 2 is a flowchart of determining a carbohydrate factor, according to an embodiment of the present invention.
FIG. 3 is a flowchart of determining a correction factor, according to an embodiment of the present invention.
FIGs. 4a-4d illustrate exemplary interface screens of the meter of FIG. 1.
FIG. 5 illustrates an exemplary interface screen of the meter of FIG. 1.

While a given embodiment is susceptible to various modifications and alternative forms, specific embodiments have been shown by way of example in the drawings and will be described in detail herein. It should be understood, however, that the invention is not intended to be limited to the particular forms disclosed. Rather, the spirit and scope of the invention, the protection of which is defined by the appended claims, are intended to cover all modifications, equivalents, and alternatives of the details described herein.

## DESCRIPTION OF ILLUSTRATED EMBODIMENTS

Existing medical device systems and glucose management devices including insulin bolus calculators suffer from a number of significant limitations. In particular, for example, such systems require diabetic individuals to input user-specific parameters such as a carbohydrate factor, an exercise adjustment factor, and a correction factor. Often, however, a diabetic individual and/or the healthcare professionals may not know the individual's carbohydrate factor and/or the correction factor. If they know the equations to calculate those factors, they can do so (often with the assistance of a calculator). However, if they do not know the equations to determine the factors, they will have to look up the equations externally and then determine the factors, which can be time consuming, inconvenient, and prone to error.

According to aspects of the present disclosure, systems, and methods are described for improving insulin bolus calculations. The described systems and methods assist with simplifying and/or removing certain steps required for determining a bolus calculation. In particular, aspects of the present invention assist in determining a carbohydrate factor, a correction factor, or both based on the individual's response to several lifestyle questions, the answers to which are generally known/memorized by the individual (and, as such, generally do not require any looking up or researching the answers).

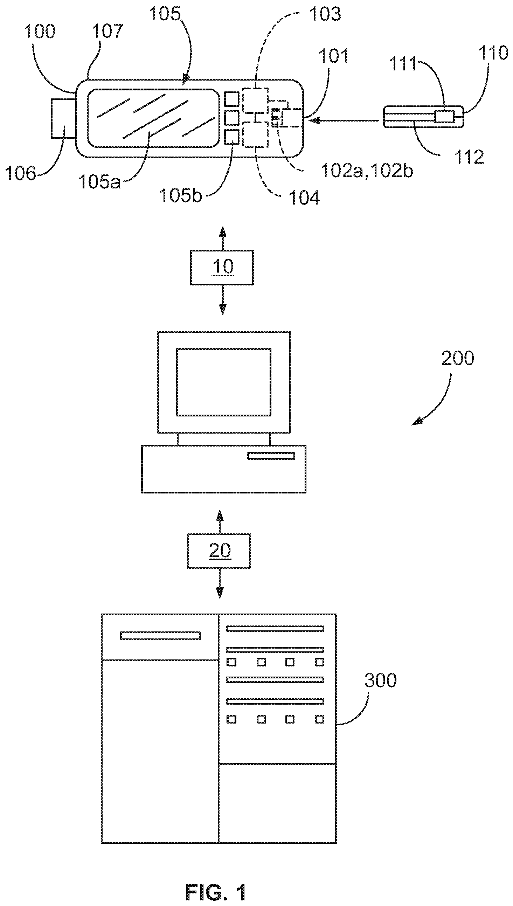

Referring to FIG. 1, a blood glucose meter 100 is described to illustrate aspects of the present invention. As shown in FIG. 1, the meter 100 includes a housing 107 having a test sensor port or opening 101 formed therein. A test sensor 110 is received into the test sensor port 101. The test sensor 110 is configured to receive a fluid (e.g., blood) sample, which has an analyte (e.g., glucose) that is to be analyzed by the meter 100. In turn, the meter 100 provides point-in-time measurements of blood glucose concentrations in blood samples received on the test sensor 110.

The test sensors described herein may be electrochemical test sensors or optical test sensors. The test sensor 110 shown in FIG. 1 is an electrochemical test sensor. As such, the test sensor 110 includes a receiving area 111 that contains a reagent that reacts with the sample to provide information relating to an analyte in the sample, i.e., blood glucose concentration. Specifically, the reagent converts the glucose in the sample into a chemical species that is electrochemically measurable and reflects the glucose concentration in the sample. The test sensor 110 also includes a plurality of electrodes 112 that transmits the measurable electrical signal from the electrochemical reaction.

Correspondingly, the meter 100 includes contacts 102a, 102b that contact the electrodes 112 on the test sensor 110 to receive the electrical signal from the electrodes 112. The meter 100 employs a processing component 103 to process the electrical signal and determine a glucose concentration measurement. The processing component 103, for example, may include an analog front end that interfaces with the contacts 102a, 102b to receive an analog signal from the test sensor 110 and a back end digital engine to digitally process the signal. The processing component 103 includes one or more computer processors that execute programmed instructions according to a measurement algorithm. The programmed instructions are stored in, and read from, at least one memory 104. The memory 104, for example, may include any type or combination of computer readable and writable storage devices. For example, the memory 104 may be a non-volatile memory, such as flash memory, or the like.

In general, the processing component 103 may execute programmed instructions stored as data on the memory 104. The programmed instructions provide various functions for the meter 100 and control various aspects of the operation of the meter 100. For example, the housing 107 of the meter 100 includes a user interface 105. The user interface 105 includes a display 105a and a user-input mechanism 105b. As discussed in more detail below, the display 105a may present information relating to the test results, the testing procedure, etc., as well as other responses to user inputs, etc. The display 105a may be a graphic liquid crystal display (LCD), an organic light-emitting diode (OLED), segment LCD, or the like. Accordingly, the processing component 103 may execute programmed instructions to show information on the display 105a. The display 105a may be a high-resolution, rich viewing display, which may present both static and moving text and images to the user. However, other types of displays, including, for example, lower resolution, monochromatic LCD displays, may be employed. In general, a range of display types, from a low-cost basic display to a fully functional display, may be employed. The display 105a may be of any suitable size. In some cases, the display 105a may cover one entire side of the instrument 100. Moreover, the display 105a may include a touchscreen. In addition, the user interface 105 may provide advanced graphical user display and audio capabilities available directly on the instrument 100 or via a communications interface with the instrument 100. The user-input mechanism 105b allows the user to interact with the instrument 100 and may include push buttons, a scroll wheel, touch-screen elements, or any combinations thereof.

The memory 104 may also store program parameters, constants, lookup tables, etc., that are employed by the processing component 103 when executing the programmed instructions. The program parameters, for example, may alter operation of the meter 100 according to geographical or market considerations. In general, the memory 104 stores software, firmware, and other data that are used for the operation of the meter 100.

As shown in FIG. 1, the meter 100 can be communicatively coupled to an external computing device 200 via a wired or wireless connection 10. The computing device 200 may be a desktop or laptop personal computer, a handheld or pocket personal computer, a computer tablet, a smart phone/device, a personal data assistant (PDA), or any other device that includes processing capabilities and other features that can be employed with the meter 100. Although the meter 100 can run a testing procedure to produce test results and present related information on the display 105a, the computing device 200 can provide more advanced functionality for managing, processing, and displaying test results and related information. For example, the computing device 200 has the necessary processing power, program memory (e.g., RAM), and display capabilities to execute health data management software that provides more advanced analysis and presentation of the test results measured by the meter 100. For instance, the computing device 200 may download test results from the meter 100, perform complex statistical analysis on the test results, and display the statistical analysis as graphs on a high-resolution display provided by the computing device 200. Alternatively, the meter 100 may be integrated with the computing device 200 to create a single device (not shown) that has the capabilities of both, such as for use in hospitals and other sophisticated environments, in which the benefits of the feature-rich functionality offset the increased costs of a more complex handheld device. Both meter 100 and computing device 200 can be characterized as a glucose management device.

Aspects of the invention assist with calculating an amount of insulin bolus for returning a user's blood glucose levels to within a desired range , or maintaining it within the desired range. In one embodiment, the processing component 103 executes programmed instructions pertaining to a bolus calculator stored on the memory 104. To calculate the bolus, the display 105a prompts the user to input short-term parameters that depend on the user's current levels of carbohydrates and insulin and his or her exercise status. The display 105a also prompts the user to (1) input user-specific parameters, including an exercise adjustment factor, a carbohydrate ("carb") factor, and a correction factor or (2) select the option for using the meter 100 to assist in calculating one or more of the user-specific parameters. If the user chooses the latter option, the processing component 103 causes the display 105a to display a series of questions (stored in the memory 104), the answers to which are commonly known to a person with diabetes and/or the person's healthcare professional. The bolus calculation also utilizes the user's insulin type (e.g., rapid-acting insulin, regular insulin, etc.), which the user is also required to input into the instrument 100.

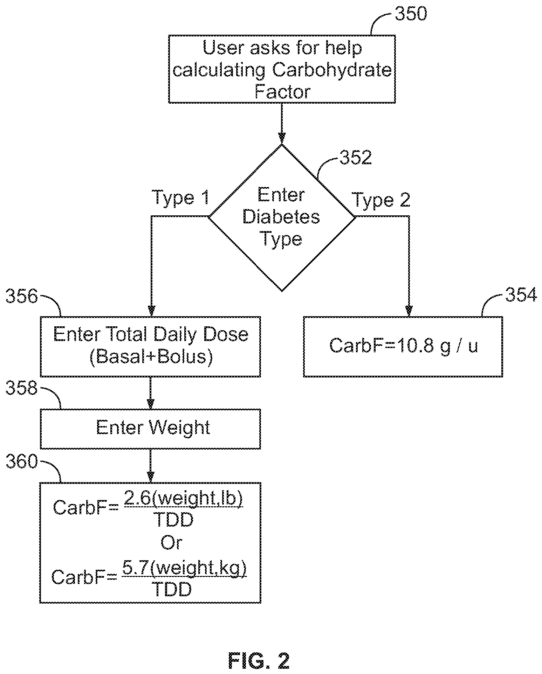

FIG. 2 is a flowchart of an exemplary method of determining a carb factor, according to one embodiment. The instrument 100 may display on the display 105a a prompt for the user to, via the user-input mechanism 105b, (1) input the user's carb factor or (2) initiate a carb factor determination sequence to obtain assistance from the meter 100 in determining the user's carb factor. At step 350, using the user-input mechanism 105b, the user selects the option for assistance in determining the carb factor. In response thereto, the processing component 103 causes the instrument 100 to run programmed instructions stored in the memory 104 pertaining to a carb factor determination sequence. The programmed instructions include causing the display 105a to display at least one query. The at least one query may include a prompt for the user to input his or her diabetes type, i.e., Type 1 or Type 2, at step 352. In other embodiments, the user's diabetes type may have been previously input by the user and stored in the memory 104.

If the user has Type 2 diabetes, the processor may determine the carb factor, via the at least one processing component 103, using information (e.g., data, an equation, or an algorithm) stored in the memory 104. The carb factor may optionally be displayed on the display 105a. In the illustrated example of FIG. 2, the carb factor is 10.8 g/U, as shown in step 354. However, this value may vary, as it is generally unique to the individual. In one embodiment, the value may range from about 1 to about 200. In another embodiment, the value may range from about 6 to about 30.

If, the user has Type 1 diabetes, the at least one query further includes a query associated with the user's total daily doses (TDD) of basal insulin and bolus insulin, a query associated with the user's weight, or the combination thereof. Basal insulin doses are baseline amounts of insulin needed to keep blood glucose levels at consistent levels during periods of fasting. Bolus insulin doses are amounts of insulin needed to process digested sugars and carbohydrates due to the increased need for insulin after meals. Referring back to FIG. 2, the display 105a displays a prompt for the user to enter the user's TDD at step 356. The display 105a may then display a prompt for the user to enter his or her weight at step 358. It is contemplated that the weight prompt (step 358) may come before the TDD prompt (step 356) or that the user's weight may have been previously entered and stored in the memory 104. The processing component 103 may then determine the carb factor at step 360 using information (e.g., data, an equation, or an algorithm) stored in the memory 104. The carb factor may optionally be displayed on the display 105a. In the illustrated embodiment, the carb factor is (2.6 x weight (lb))/TDD or (5.7 x weight (kg))/TDD. In another nonlimiting embodiment, the equation for calculating the carb factor is 500/TDD. It is contemplated, however, that other equations may be used.

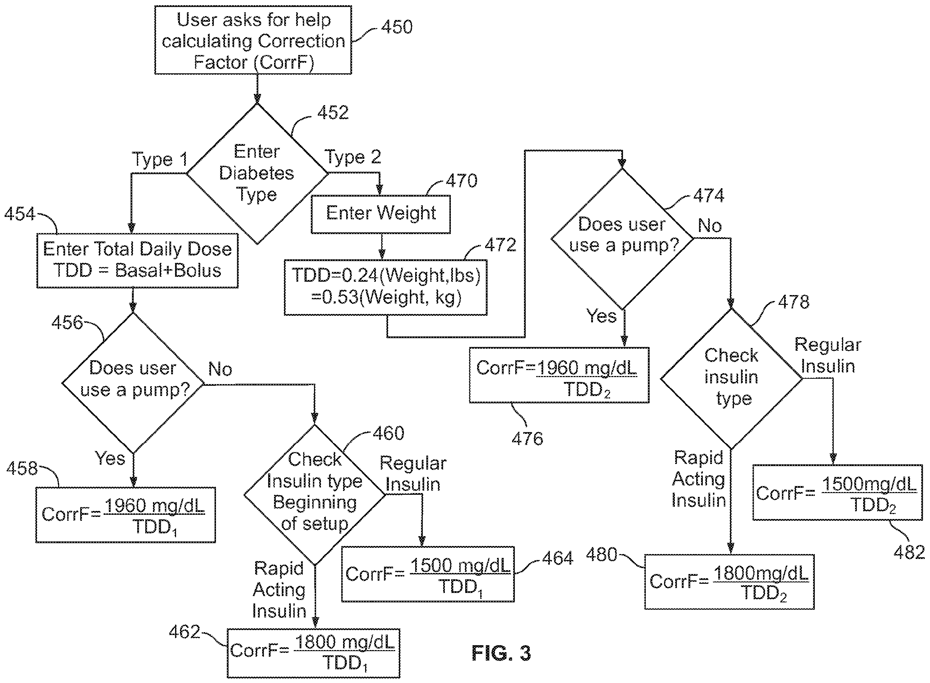

FIG. 3 is a flowchart of an exemplary method for determining a correction factor, according to one embodiment. It is contemplated that the correction factor determination sequence of FIG. 3 may be displayed and run prior to, instead of, or after the carbohydrate factor determination sequence of FIG. 2. The instrument 100 may display on the display 105a a prompt for the user to, via the user-input mechanism 105b, (1) input the user's correction factor or (2) initiate a correction factor determination sequence to obtain assistance from the meter 100 in determining the user's correction factor. At step 450, using the user-input mechanism 105b, the user selects the option for assistance in determining the correction factor. In response thereto, the processing component 103 causes the instrument 100 to run programmed instructions stored in the memory 104 pertaining to a correction factor determination sequence. The programmed instructions include causing the display 105a to display at least one query. The at least one query may include a prompt for the user to input the user's diabetes type (i.e., Type 1 or Type 2), the user's insulin pump usage, or the like. In step 452 of FIG. 3, the user is prompted to enter the user's diabetes type. In other embodiments, the user's diabetes type may have been previously input and stored in the memory 104. If the user inputs that the user has Type 1 diabetes, the display 105a displays a query associated with the user's total daily doses ($TDD_1$) of basal insulin and bolus insulin. At step 454, the display 105a queries the user to enter the user's $TDD_1$.

At step 456, the display 105a queries the user to input whether or not the user uses an insulin pump. In other embodiments, information regarding whether the user uses an insulin pump may have been previously entered and stored in the memory 104, in which case step 456 may be omitted. In the illustrated embodiment, if an insulin pump is used (step 458), the processing component 103 may determine the correction factor using information (e.g., data, an equation, or an algorithm) stored in the memory 104. In the illustrated embodiment, the correction factor is determined to be (1960 mg/dL)/$TDD_1$, which may optionally be displayed on the display 105a. It is contemplated, however, that other equations may be used.

If an insulin pump is not used, the processing component 103 checks for the type of insulin injected by the user (step 460), which may be stored by the memory 104. In some embodiments, the user is prompted to enter the type of insulin used during the correction factor determination sequence shown in FIG. 3. If the insulin type is rapid-acting insulin (e.g., insulin that begins to work about 15 minutes after injection, peaks after about 1 hour, and is effective for about 2-4 hours) (step 462), the correction factor is determined using data, an algorithm, and/or an equation stored in the memory. In the illustrated embodiment, the processing component 103 has determined the correction factor to be (1800 mg/dL)/$TDD_1$. It is contemplated, however, that other equations may be used. If the insulin type is regular insulin (e.g., insulin that usually reaches the bloodstream within about 30 minutes after injection, peaks between 2-3 hours after injection, and is effective for about 3-6 hours) (step 464), the correction factor is determined using data, an algorithm, and/or an equation stored in the memory 104. In the illustrated embodiment, the processing component 103 has determined the correction factor to be (1500 mg/dL)/$TDD_1$. It is contemplated, however, that other equations may be used.

If the user has Type 2 diabetes, the display 105a may display a prompt for the user to enter the user's weight at step 470. It is contemplated that user's weight may have been previously stored in the memory 104, in which case step 470 may be omitted. The processing component 103 then determines, at step 472, the effective total daily doses ($TDD_2$) of basal insulin and bolus insulin at step 472. In the illustrated embodiment, the $TDD_2$ is (0.24 x weight (lbs)) or (0.53 x weight (kg)). It is contemplated, however, that other equations may be used. At step 474, the display 105a displays a prompt for the user to input whether or not the user uses an insulin pump. In other embodiments, information regarding whether the user uses an insulin pump was previously entered and is stored in the memory 104, in which case step 474 may be omitted. In the illustrated embodiment, if an insulin pump is used (step 476), the processing component 103 determines the correction factor to be (1960 mg/dL)/$TDD_2$. As discussed above, it is contemplated that other equations may be used. If an insulin pump is not used, the processing component 103 checks for the type of insulin injected by the user (step 478), which may be stored by the memory 104. In some embodiments, the user is prompted to enter the type of insulin used during the correction factor determination sequence shown in FIG. 3. In the illustrated embodiment, if the insulin type is rapid-acting insulin (step 480), the correction factor is determined by the processing component 103 as (1800 mg/dL)/$TDD_2$. In the illustrated embodiment, if the insulin type is regular insulin (step 482), the correction factor is determined by the processing component 103 as (1500 mg/dL)/$TDD_2$. As discussed above, it is contemplated that other equations may be used.

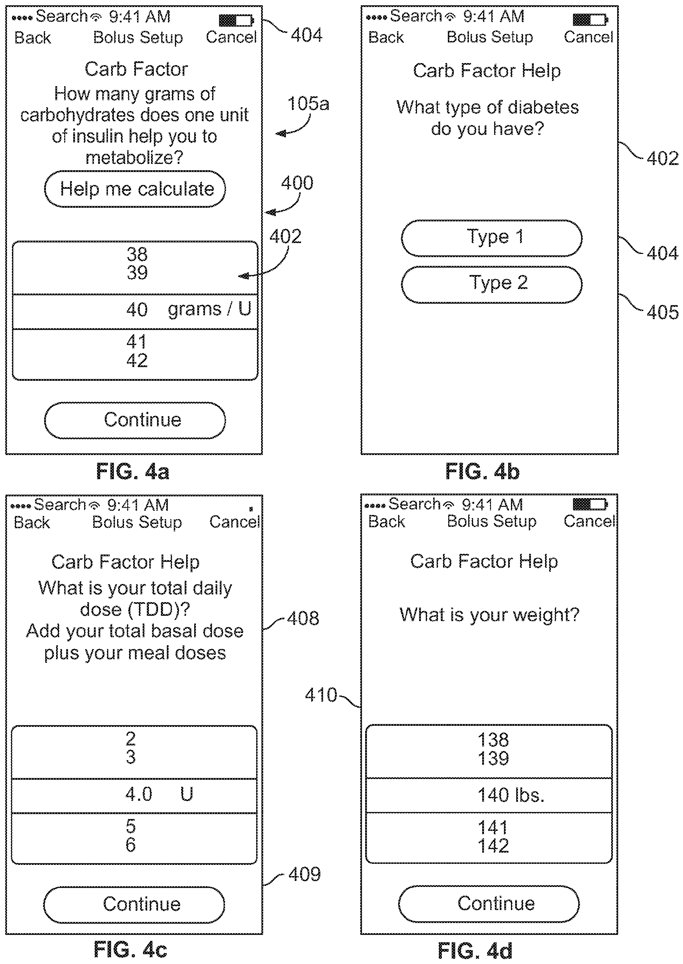

Referring to FIGs. 4a-d, interface screens, displayed on the display 105a, associated with a carb factor determination are shown according to one embodiment. The interface screens shown and described herein may also or alternatively appear, for example, on the computing device 200. As shown in FIG. 4a, the user is prompted, via the interface screen 400, to enter the user's carb factor, i.e., how many grams of carbohydrates one unit of insulin helps the user to metabolize. The user may make a selection from a series of carbohydrate amounts provided on a segment 402 of the interface screen 400 using a scrolling feature, touching/swiping the display 105a, or the like. In other embodiments, the user may manually enter the appropriate amount of carbohydrates via the user-input mechanism 105b. If the carb factor is unknown, the user may select the "Help me calculate this" option 404 (see step 350 of FIG. 2). In FIG. 4b, a subsequent (or, in other embodiments, preceding) interface screen 402 displays the query "What type of diabetes to you have?" corresponding with step 352 of FIG. 2. The user may select from "Type 1" (404) or "Type 2" (405). In FIG. 4c, in response to a user selecting Type 1 (404), a subsequent interface screen 408 displays the query "What is your total daily dose (TDD)? Add your total basal dose plus your meal doses," corresponding with step 356 of FIG. 2. The user may select the appropriate TDD and then select "Continue" (409). FIG. 4d illustrates an interface screen 410 prompting the user to enter the user's weight, corresponding with step 358 of FIG. 2.

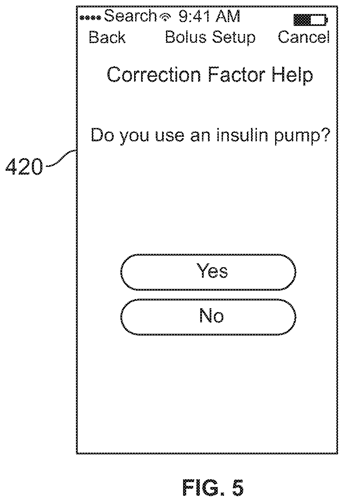

FIG. 5 illustrates one example of an interface screen 420 corresponding with the correction factor determination (see FIG. 3). The interface screen 420 corresponds with step 456 or step 474 of FIG. 3 and queries, "Do you use an insulin pump?"

It is contemplated that the exemplary methods shown in FIGs. 2 and 3 may be modified as appropriate. For example, the order of the queries displayed on the display may be altered. It is contemplated that more or other information may be queried of the user and that, accordingly, interface screens other than those shown in FIGs. 4a-4d and 5 may be displayed on the display 105a. It is also contemplated that less information may be queried of a user, especially if certain information has already been entered by the user and stored in the meter memory 104.

The carbohydrate factor and/or the correction factor ascertained by the meter according to the embodiments discussed herein may then be used to calculate an amount of bolus insulin needed by the user to return his or her blood glucose to within a normal range. The bolus insulin amount is determined using the user's current blood glucose value, carbohydrate amount, amount of exercise, insulin amount, target blood glucose value/range, carb factor, correction factor, and exercise factor.

While the invention is susceptible to various modifications and alternative forms, specific embodiments and methods thereof have been shown by way of example in the drawings and are described in detail herein. It should be understood, however, that it is not intended to limit the invention to the particular forms or methods disclosed, but, to the contrary, the intention is to cover all modifications, equivalents and alternatives falling within the scope of the claim.

**Claims**

1. A glucose management device for managing a user's blood glucose level, the glucose management device comprising:

   a housing (107);
   a user interface (105) including a display (105a) and a user-input mechanism;
   a memory device for storing programmed instructions and data; and
   a processing component (103), the processing component (103) configured to

   (a) causing the display (105a) to display a prompt for input via the user-input mechanism, wherein the input includes:

   - whether a type of diabetes is type 1 or type 2, if not stored in the memory device
   - whether a pump is used, if not stored in the memory device,
   - whether an insulin type used is regular insulin or rapid acting insulin;
   - a user's weight, if not stored in the memory device;

   wherein
   (a1) in case of type 1 diabetes,
   input further includes a total daily dose ($TDD_1$) consisting of basal and bolus insulin; and
   the carbohydrate factor (CarbF) is calculated by the formula

   $$CarbF = 2.6 \times \text{weight (lbs)} / TDD_1 \text{ or } 5.7 \times \text{weight (kg)} / TDD_1;$$

   (a2) in case of type 2 diabetes,
   the total daily dose ($TDD_2$) is calculated by the formula

   $$TDD_2 = 0.24 \times \text{weight (lbs) or } 0.53 \times \text{weight (kg)},$$

   and the carbohydrate factor (CarbF) is a constant value retrieved from the memory device;
   (b) calculating the correction factor (CorrF) as follows:

   if a pump is used: CorrF = 1960 mg/dL / TDD,
   if no pump is used and the insulin type is regular insulin: CorrF = 1500 mg/dL / TDD
   if no pump is used and the insulin type is rapid acting insulin: CorrF = 1800 mg/dL / TDD,

   wherein TDD = $TDD_1$ or $TDD_2$, respectively;
   and
   (c) determining an amount of bolus insulin using the carbohydrate factor and the correction factor.

**Patentansprüche**

1. Blutzucker-Verwaltungsgerät zum Verwalten des Blutzuckerspiegels eines Benutzers, wobei das Blutzucker-Verwaltungsgerät umfasst:

   ein Gehäuse (107);
   eine Benutzerschnittstelle (105) mit einer Anzeige (105a) und einem Benutzereingabemechanismus;
   eine Speichervorrichtung zum Speichern von programmierten Befehlen und Daten; und
   eine Verarbeitungskomponente (103), wobei die Verarbeitungskomponente (103) dazu eingerichtet ist,

   (a) die Anzeige (105a) zu veranlassen, eine Aufforderung zur Eingabe über den Benutzereingabemechanismus anzuzeigen,
   wobei die Eingabe enthält:

- ob ein Diabetes-Typ Typ 1 oder Typ 2 ist, falls nicht in der Speichervorrichtung gespeichert
- ob eine Pumpe verwendet wird, falls nicht in der Speichervorrichtung gespeichert
- ob ein verwendeter Insulin-Typ reguläres Insulin oder schnell wirkendes Insulin ist;
- das Gewicht eines Benutzers, falls nicht in der Speichervorrichtung gespeichert;

wobei
(a1) im Falle von Typ 1-Diabetes
Eingabe ferner eine Gesamttagesdosis ($TDD_1$) enthält, die aus Basal- und Bolusinsulin besteht; und
der Kohlenhydratfaktor (CarbF) nach folgender Formel berechnet wird

$$CarbF = 2,6 \text{ x Gewicht (lbs)} / TDD_1 \text{ oder } 5,7 \text{ x Gewicht (kg)} / TDD_1;$$

(a2) im Falle von Typ 2-Diabetes,
die Gesamttagesdosis ($TDD_2$) nach folgender Formel berechnet wird

$$TDD_2 = 0,24 \text{ x Gewicht (lbs)} \text{ oder } 0,53 \text{ x Gewicht (kg)},$$

und der Kohlenhydratfaktor (CarbF) ein konstanter Wert ist, der aus der Speichervorrichtung abgerufen wurde;
(b) den Korrekturfaktor (CorrF) wie folgt zu berechnen:
wenn eine Pumpe verwendet wird: CorrF = 1960 mg/dL / TDD,
wenn keine Pumpe verwendet wird und der Typ des Insulins reguläres Insulin ist: CorrF = 1500 mg/dL / TDD
wenn keine Pumpe verwendet wird und der Typ des Insulins schnell wirkendes Insulin ist: CorrF = 1800 mg/dL / TDD,
wobei TDD = $TDD_1$ bzw. $TDD_2$ ist;
und
(c) eine Bolusinsulinmenge unter Verwendung des Kohlenhydratfaktors und des Korrekturfaktors zu bestimmen.

**Revendications**

1.  Appareil déterminant la dose de glucose pour la gestion de glucose sanguin d'un utilisateur, l'appareil déterminant le dose de glucose comprenant :

    un boîtier (107) ;
    une interface utilisateur (105) comprenant un affichage (105a) et un mécanisme d'entrée utilisateur ;
    un dispositif de mémoire pour stocker des instructions et des données programmées ; et
    un composant de traitement (103), le composant de traitement (103) étant configuré pour

    (a) faire en sorte que l'affichage (105a) affiche une invite pour une entrée via le mécanisme d'entrée de l'utilisateur,
    dans lequel l'entrée comprend :

    - si un type de diabète est le type 1 ou le type 2, s'il n'est pas stocké dans le dispositif de mémoire
    - si une pompe est utilisée, si elle n'est pas stockée dans le dispositif de mémoire,
    - si un type d'insuline utilisé est l'insuline régulière ou l'insuline à action rapide ;
    - le poids de l'utilisateur, s'il n'est pas stocké dans le dispositif de mémoire ;

    dans lequel
    (a1) en cas de diabète de type 1,
    l'entrée comprend en outre une dose quotidienne totale ($TDD_1$) constituant d'insuline basale et d'glucose sanguin ; et
    le facteur glucidique (CarbF) est calculé par la formule

$$CarbF = 2{,}6 \times poids\ (lbs)\ /\ TDD_1\ ou\ 5{,}7 \times poids\ (kg)\ /\ TDD_1\ ;$$

(a2) en cas de diabète de type 2,
la dose journalière totale ($DTT_2$) est calculée par la formule suivante

$$TDD_2 = 0{,}24 \times poids\ (lbs)\ ou\ 0{,}53 \times poids\ (kg),$$

et le facteur glucidique (CarbF) est une valeur constante extraite du dispositif de mémoire ;
(b) calculer le facteur de correction {CorrF) comme suit :
si une pompe est utilisée : CorrF = 1960 mg/dL / TDD,
si aucune pompe n'est utilisée et que le type d'insuline est de l'insuline ordinaire : CorrF = 1500 mg/dL / TDD. si aucune pompe n'est utilisée et que le type d'insuline est une insuline à action rapide : CorrF = 1800 mg/dL/TDD,
dans lequel TDD = $TDD_1$ ou $TDD_2$, respectivement ;
et
(c) déterminer une quantité de glucose sanguin en utilisant le facteur glucidique et le facteur de correction.

**FIG. 1**

350

User asks for help
calculating Carbohydrate
Factor

352

Type 1  Enter
Diabetes
Type  Type 2

356

Enter Total Daily Dose
(Basal+Bolus)

354

CarbF=10.8 g / u

358

Enter Weight

360

$$CarbF= \frac{2.6(weight,lb)}{TDD}$$

Or

$$CarbF= \frac{5.7(weight,kg)}{TDD}$$

**FIG. 2**

**FIG. 3**

- User asks for help calculating Correction Factor (CorrF) — 450
- Enter Diabetes Type — 452
  - Type 1 → Enter Total Daily Dose TDD = Basal+Bolus — 454
    - Does user use a pump? — 456
      - Yes → $CorrF = \dfrac{1960\ mg/dL}{TDD_1}$ — 458
      - No → Check Insulin type Beginning of setup — 460
        - Rapid Acting Insulin → $CorrF = \dfrac{1800\ mg/dL}{TDD_1}$ — 462
        - Regular Insulin → $CorrF = \dfrac{1500\ mg/dL}{TDD_1}$ — 464
  - Type 2 → Enter Weight — 470
    - $TDD = 0.24(Weight, lbs) = 0.53(Weight, kg)$ — 472
    - Does user use a pump? — 474
      - Yes → $CorrF = \dfrac{1960\ mg/dL}{TDD_2}$ — 476
      - No → Check insulin type — 478
        - Rapid Acting Insulin → $CorrF = \dfrac{1800\ mg/dL}{TDD_2}$ — 480
        - Regular Insulin → $CorrF = \dfrac{1500\ mg/dL}{TDD_2}$ — 482

EP 3 295 342 B1

## FIG. 4a

•••• Search 🛜 9:41 AM     🔋

Back    Bolus Setup    Cancel    ⟵ 404

### Carb Factor

How many grams of carbohydrates does one unit of insulin help you to metabolize?

( Help me calculate )    ⟵ 105a

   ⟵ 400

   ⟵ 402

| |
|---|
| 38 |
| 39 |
| 40    grams / U |
| 41 |
| 42 |

( Continue )

**FIG. 4a**

## FIG. 4b

•••• Search 🛜 9:41 AM     🔋

Back    Bolus Setup    Cancel

### Carb Factor Help

What type of diabetes do you have?

   ⟵ 402

( Type 1 )    ⟵ 404

( Type 2 )    ⟵ 405

**FIG. 4b**

## FIG. 4c

•••• Search 🛜 9:41 AM     ▪

Back    Bolus Setup    Cancel

### Carb Factor Help

What is your total daily dose (TDD)?
Add your total basal dose plus your meal doses

   ⟵ 408

| |
|---|
| 2 |
| 3 |
| 4.0    U |
| 5 |
| 6 |

   ⟵ 409

( Continue )

**FIG. 4c**

## FIG. 4d

•••• Search 🛜 9:41 AM     🔋

Back    Bolus Setup    Cancel

### Carb Factor Help

What is your weight?

   410 ⟵

| |
|---|
| 138 |
| 139 |
| 140 lbs. |
| 141 |
| 142 |

( Continue )

**FIG. 4d**

420 —

•••• Search 📶 9:41 AM 🔋
Back        Bolus Setup      Cancel

Correction Factor Help


Do you use an insulin pump?




( Yes )

( No )

**FIG. 5**

**EP 3 295 342 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012245447 A1

- US 2009253970 A1

**Non-patent literature cited in the description**

- **JOHN WALSH et al.** Guidelines for Optimal Bolus Calculator Settings in Adults. *Journal of Diabetes Science and Technology Volume Diabetes Technology Society J Diabetes Sci Technol,* 01 January 2011, 129-135, http://www.ncbi.nlm.nih.gov/pmc/articles/PMC3045234/pdf/dst-05-129.pdf

- **MAGUIRE, PHYLLIS.** Tips for calculating a total daily dose of insulin. *Today's Hospitalist,* 02 April 2015, 2-3, https://web.archive.org/web/20150402005842/http://www.todayshospitalist.com/articles/tips-for-calculating-a-daily-dose-of-insulin.htm